(19) Europäisches Patentamt
European Patent Office
Office européen des brevets

(11) Veröffentlichungsnummer : **0 348 833 B1**

# (12) EUROPÄISCHE PATENTSCHRIFT

(45) Veröffentlichungstag der Patentschrift : **05.08.92 Patentblatt 92/32**

(51) Int. Cl.$^5$ : **C01G 55/00,** C22B 3/00

(21) Anmeldenummer : **89111444.9**

(22) Anmeldetag : **23.06.89**

## (54) Verfahren zur Abtrennung und Wiedergewinnung von Rhodium aus den Produkten der Oxosynthese.

(30) Priorität : **30.06.88 DE 3822037**

(43) Veröffentlichungstag der Anmeldung : **03.01.90 Patentblatt 90/01**

(45) Bekanntmachung des Hinweises auf die Patenterteilung : **05.08.92 Patentblatt 92/32**

(84) Benannte Vertragsstaaten : **AT DE ES FR GB SE**

(56) Entgegenhaltungen :
**EP-A- 0 015 379**
**EP-A- 0 147 824**
**EP-A- 0 156 253**
**DE-A- 2 448 005**

(56) Entgegenhaltungen :
**DE-A- 3 443 474**
**GB-A- 2 085 747**
**US-A- 3 547 964**
**US-A- 4 400 547**

(73) Patentinhaber : **HOECHST AKTIENGESELLSCHAFT**
**Postfach 80 03 20**
**W-6230 Frankfurt am Main 80 (DE)**

(72) Erfinder : **Lappe, Peter, Dr. Dipl.-Chem.**
**Eickenhof 34**
**W-4220 Dinslaken (DE)**
Erfinder : **Bexten, Ludger, Dr. Dipl.-Chem.**
**Im Freihof 9**
**W-4224 Hünxe (DE)**
Erfinder : **Kupies, Dieter**
**Burgfeld 143**
**W-4100 Duisburg (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein verbessertes Verfahren zur Abtrennung und Rückgewinnung von Rhodium aus den Reaktionsgemischen der Oxosynthese.

Die Herstellung von Aldehyden und Alkoholen durch Anlagerung von Kohlenmonoxid und Wasserstoff an olefinische Doppelbindungen (Hydroformylierung) ist bekannt. Die Reaktion wird durch Metalle der 8. Nebengruppe des Periodensystems oder deren Verbindungen katalysiert, die unter den Reaktionsbedingungen Carbonyle oder Hydridocarbonyle bilden. Während früher fast ausschließlich Kobalt und Kobaltverbindungen als Katalysatoren eingesetzt wurden, finden heute in zunehmendem Maße Rhodiumkatalysatoren Anwendung, obgleich Rhodium um ein vielfaches teurer als Kobalt ist. Rhodium gelangt dabei allein oder in Kombination mit Komplexbildnern, z.B. organischen Phosphinen, zum Einsatz. Während die Oxosynthese mit Rhodium als Katalysator Reaktionsdrücke von 25 bis 30 MPa erfordert, genügen bei Einsatz von Rhodium-Komplexverbindungen Drücke von 1 bis 5 MPa.

Für Rhodium-Katalysatoren ergeben sich in vielen Fällen deutliche Vorteile. Sie besitzen höhere Aktivität und Selektivität und ermöglichen überdies einen komplikationsfreien Betrieb der Produktionsanlage, insbesondere hinsichtlich der Durchführung der Synthese und der Ausbringung der Produkte aus dem Reaktor. Schließlich kann das klassische Oxoverfahren auf Basis von Kobaltkatalysatoren unter Verwendung der vorhandenen Apparateteile in vielen Fällen mit nur geringen Investitionen auf Rhodiumkatalysatoren umgestellt werden.

Erhebliche Schwierigkeiten bereitet jedoch die verlustfreie oder zumindest annähernd verlustfreie Abtrennung und Wiedergewinnung des Rhodiums, unabhängig davon, ob es mit oder ohne zusätzlichem Komplexbildner als Katalysator eingesetzt wird. Nach Beendigung der Umsetzung findet sich das Rhodium als Carbonylverbindung, die gegebenenfalls noch weitere Liganden enthalten kann, gelöst im Hydroformylierungsprodukt.

Zur Aufarbeitung wird das Oxorohprodukt üblicherweise mehrstufig entspannt, indem man den Synthesedruck, der je nach Art des eingesetzten Rhodiumkatalysators etwa 1 bis 30 MPa beträgt, zunächst auf etwa 0,5 bis 2,5 MPa reduziert. Hierbei wird im Rohprodukt gelöstes Synthesegas frei. Anschließend kann man auf Normaldruck entspannen. Die Abtrennung des Rhodiums erfolgt entweder unmittelbar aus dem Rohprodukt oder aus dem Rückstand der Rohproduktdestillation. Der erste Weg wird dann beschritten, wenn in der vorausgegangenen Hydroformylierungsstufe Rhodium ohne zusätzlichen Komplexbildner als Katalyator eingesetzt worden war. Die zweite Variante wird angewandt, wenn der Rhodiumkatalysator außer Kohlenmonoxid noch weitere Liganden, z.B. Phosphine oder Phosphite in komplexer Bindung enthält. Sie kann auch dann genutzt werden, wenn die Hydroformylierung zwar mit Rhodium allein durchgeführt, dem Rohprodukt nach Entspannung aber ein Komplexbildner zur Stabilisierung des Rhodiums zugesetzt worden war. Grundsätzlich ist zu berücksichtigen, daß das Edelmetall im Rohprodukt in einer Konzentration von nur wenigen ppm vorliegt, seine Abtrennung daher sehr sorgfältiges Arbeiten erfordert. Zusätzliche Schwierigkeiten können dadurch entstehen, daß das Rhodium, insbesondere wenn es ohne Ligand eingesetzt wurde, bei der Entspannung teilweise in metallische Form übergeht oder mehrkernige Carbonyle bildet. Es kommt dann zur Ausbildung eines heterogenen Systems, das aus der flüssigen organischen und der festen, Rhodium oder Rhodiumverbindungen enthaltenden Phase besteht.

Für die Abtrennung von Rhodium aus dem Oxorohprodukt sind mehrere Verfahren bekannt. Nach der Arbeitsweise der DE 33 47 406 A1 gewinnt man Rhodium durch Extraktion mit komplexbildenden Reagenzien aus dem Oxorohprodukt wieder. Nach einer bevorzugten Ausführungsform setzt man als Komplexbildner Sulfonate und Carboxylate organischer Phosphine ein.

Besonders zweckmäßige komplexbildende Reagenzien sind die sulfonierten Triphenylphosphine, z.B. Salze der Triphenylphosphin-trisulfonsäure.

Die aus Rhodium und den Sulfonaten oder Carboxylaten der organischen Phosphine gebildeten Komplexverbindungen sind wasserlöslich. Dementsprechend kann das Rhodium aus dem Oxorohprodukt, also der organischen Phase, mit einer wäßrigen Lösung des substituierten Phosphins extrahiert werden. Die wäßrige, Rhodium enthaltende Phase, läßt sich durch einfaches Dekantieren vom organischen Produktgemisch abtrennen. Durch Kreislaufführung können in der Lösung des Komplexbildners hohe Rhodium-Konzentrationen erreicht werden.

Um die Extraktion des Rhodiums aus der organischen Phase und seinen Übergang in die wäßrige Phase zu beschleunigen und zu vervollständigen, setzt man nach der DE 34 11 034 A1 der wäßrigen Lösung des Komplexbildners einen Lösungsvermittler zu.

Seine Wirkung besteht insbesondere darin, daß er die physikalischen Eigenschaften der Grenzflächen zwischen den beiden flüssigen Phasen verändert. Dadurch wird der Übergang des wäßrigen Extraktionsmittels in die Produktphase und des Rhodiums aus der Produktphase in die wäßrige Komplexbildner-Phase beschleunigt, die Extraktion vereinfacht und der apparative Aufwand verringert.

Die Rhodium-Extraktion ist umso vollständiger, je höher die Konzentration des Lösungsvermittlers in der wäßrigen Phase ist. Seine Menge kann jedoch nicht unbegrenzt vermehrt werden, weil er die wäßrige Lösung des Extraktionsmittels belastet und seine Stabilität beeinträchtigt.

Das Verfahren der DE 34 43 474 A1 verwendet als Komplexbildner daher quartäre Ammoniumsalze sulfonierter Triphenylphosphine. Die Herstellung dieser Salze ist jedoch sehr aufwendig, so daß die Wirtschaftlichkeit des Extraktionsprozesses nicht immer gegeben ist.

Auch zur Abtrennung des Rhodiums aus den Destillationsrückständen des Oxorohproduktes sind verschiedene Prozesse bekannt.

Nach dem Verfahren der EP 00 15 379 A1 oxidiert man in der Oxosynthese eingesetzte Rhodium-Ligand-Katalysatoren in Gegenwart von Aldehyd z.B. mit Luft und entfernt die entstandenen festen Reaktionsprodukte. Die erhaltene Lösung kann nach Ergänzung von Ligand wieder als Katalysator eingesetzt werden. Der Anwendung dieser Arbeitsweise sind Grenzen dadurch gesetzt, daß die bei der Destillation anfallenden Rückstände nicht abgetrennt werden.

Gegenstand der US-PS 4 400 547 ist ein Hydroformylierungsprozeß in Gegenwart von unmodifiziertem Rhodium als Katalysator. Nach Zusatz eines Phosphorliganden wie Triphenylphosphin zur Oxorohprodukt destilliert man den Aldehyd ab. Anschließend wird der Destillationsrückstand mit Sauerstoff behandelt, um den Liganden aus der Komplexverbindung wieder abzuspalten und das Rhodium in aktiver Form zurückzugewinnen. Eine vollkommene Trennung von Rhodium und Destillationsrückstand ist nach dieser Arbeitsweise nicht möglich.

Eine Abtrennung von Edelmetallen wie Rhodium aus hochsiedenden Hydroformylierungsrückständen wird in der US-PS 3 547 964 beschrieben. Hierzu behandelt man die Rückstände in Gegenwart von Säuren wie Ameisensäure, Salpetersäure oder Schwefelsäure mit Wasserstoffperoxid. Wegen des hohen Preises von Wasserstoffperoxid und seiner problematischen Handhabung sind der technischen Anwendung des Verfahrens Grenzen gesetzt.

Nach der DE 24 48 005 C2 wird ein Rhodium enthaltender Destillationsrückstand zunächst mit Säuren und Peroxiden behandelt. Darauf zerstört man überschüssige Peroxide durch Erhitzen und setzt die das Katalysatormetall enthaltende wäßrige Lösung in Gegenwart eines wasserlöslichen organischen Lösungsmittels mit Halogenwasserstoffsäure oder Alkalihalogeniden sowie mit tertiären Phosphinen und Kohlenmonoxid oder Kohlenmonoxid abgespaltenden Verbindungen um. Diese Arbeitsweise erfordert wiederum die Verwendung von Peroxiden mit den oben geschilderten Nachteilen und den Einsatz halogenbeständiger Werkstoffe.

Es bestand daher die Aufgabe ein Verfahren zu entwickeln, mit dem es gelingt, Rhodium aus Rückständen möglichst einfach und vollständig zurückzugewinnen, die bei der Destillation von Reaktionsgemischen der Oxosynthese erhalten werden.

Erfindungsgemäß wird diese Aufgabe gelöst durch ein Verfahren zur Abtrennung und Wiedergewinnung von Rhodium aus den Destillationsrückständen von Oxosyntheseprodukten. Es ist dadurch gekennzeichnet, daß man die Destillationsrückstände zunächst mit einem Oxidationsmittel behandelt und darauf in Gegenwart von Kohlenmonoxid oder einer Kohlenmonoxid abspaltenden Verbindung mit der wäßrigen Lösung eines mit Rhodium eine wasserlösliche Komplexverbindung bildenden Reagenzes umsetzt.

Unter dem Begriff Destillationsrückstände werden die hochsiedenden Anteile der Hydroformylierungsprodukte verstanden, die nach Abdestillieren des Vorlaufs, der unter anderem Kohlenwasserstoff enthält, sowie der Aldehyde und Alkohole, zurückbleiben. Sie bestehen im wesentlichen aus den Produkten der Kondensation von Aldehyden untereinander und der Reaktion von Aldehyden mit Alkoholen. Die Destillationsrückstände enthalten darüber hinaus katalytisch wirkendes Rhodium sowie Verbindungen, die als Liganden mit dem Rhodium unter Komplexbildung reagieren. Solche Liganden sind z.B. organische Phosphine wie Triphenylphosphin. Sie sind entweder von Anfang an im Hydroformylierungsgemisch enthalten und Bestandteil des Katalysators oder sie werden nach Abschluß der Hydroformylierungsreaktion, aber vor der Destillation, dem Reaktionsgemisch zugesetzt. Ihre Aufgabe ist es in diesem Fall, die Rhodiumverbindungen gegenüber thermischer Belastung zu stabilisieren, um die Abscheidung unlöslicher Rhodiumverbindungen oder metallischen Rhodiums zu unterbinden.

Erfindungsgemäß werden die Destillationsrückstände mit einem Oxidationsmittel behandelt. Als Oxidationsmittel eignen sich insbesondere Sauerstoff oder Sauerstoff enthaltende Gase, vorzugsweise Luft. Daneben sind aber auch andere Oxidationsmittel brauchbar. Bewährt haben sich z.B. Wasserstofffperoxid und Wasserstoffperoxid bildende Verbindungen wie Natriumperoxid und ferner Hypochlorite und Permanganate. Die Oxidation erfolgt bei 20 bis 200°C, insbesondere 50 bis 150°C und vorzugsweise 80 bis 120°C. Die Anwendung von Druck ist nicht erforderlich, zur Abkürzung der Reaktionszeit aber häufig zweckmäßig. Drücke von 0,1 bis 5 MPa haben sich bewährt. Ebenso empfiehlt es sich, oxidierende Gase durch geeignete Vorrichtungen im Destillationsrückstand möglichst fein zu verteilen. Die Reaktionszeit ist von den gewählten Bedingungen abhängig. Sie beträgt bei diskontinuierlicher Arbeitsweise 0,1 bis 10 Stunden, vorzugsweise 2 bis 5

Stunden.

Der Oxidation schließt sich die Behandlung des Destillationsrückstandes mit der wäßrigen Lösung einer mit Rhodium einen Komplex bildenden Verbindung an.

Als Komplexbildner wählt man erfindungsgemäß solche Verbindungen aus, die unter den angewandten Temperaturbedingungen mit Rhodium stabilere Komplexe eingehen als Kohlenmonoxid. Dabei gilt, daß die Rhodiumabtrennung umso vollständiger ist, je größer die Neigung der komplexbildenden Reagenzien zur Reaktion mit Rhodium ist. Darüber hinaus wird gefordert, daß die Komplexverbindung in Wasser, nicht aber in organischen Medien löslich ist.

Als Komplexbildner für Rhodium bevorzugt sind Phosphorverbindungen, die zur Ausbildung von Koordinationsbindungen mit dem Edelmetall fähige Phosphoratome enthalten, d.h. Phosphoratome mit freien Elektronenpaaren. Unter diesen Verbindungen eignen sich insbesondere die Sulfonate und Carboxylate organischer Phosphine, das sind Verbindungen der allgemeinen Formel

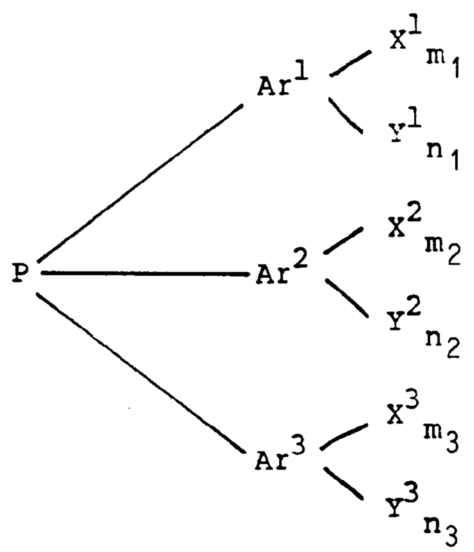

Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine geradkettige oder verzweigte Alkoxygruppe mit 1 bis 4 C-Atomen, ein Halogenatom, die OH-, CN-, $NO_2$- oder $R^1R^2N$-Gruppe, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$-) und/oder ein Sulfonat-($SO_3^-$-)Rest, $m_1$, $m_2$, $m_3$ sind gleiche oder verschiedene ganze Zahlen von 0 bis 3, wobei mindestens eine Zahl $m_1$, $m_2$, $m_3$ gleich oder größer als 1 ist; $n_1$, $n_2$, $n_3$ sind gleiche oder verschiedene ganze Zahlen von 0 bis 5. Als Kationen enthalten die vorstehend aufgeführten Carboxylate bzw. Sulfonate Alkalimetall-, Erdalkalimetall-, Zink-, Ammonium- oder quaternäre Ammoniumionen der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht.

Nach einer bevorzugten Ausführungsform des erfindungsgemässen Verfahrens verwendet man als komplexbildende Reagenzien Verbindungen der vorstehend beschriebenen allgemeinen Formel, in der $Ar^1$, $Ar^2$, $Ar^3$ jeweils einen Phenylrest, $X^1$, $X^2$, $X^3$ jeweils einen Sulfonatrest und $m_1$, $m_2$, $m_3$ jeweils die Zahl 0 oder 1 bedeuten, wobei die Summe von $m_1$, $m_2$, $m_3$ 1, 2 oder 3 ist.

Erfindungsgemäß erfolgt die Behandlung des oxidierten Destillationsrückstandes mit dem Komplexbildner in Gegenwart von Kohlenmonoxid oder einer Kohlenmonoxid abspaltenden Verbindung. Je g-Atom Rhodium im Destillationsrückstand setzt man mindestens 100 mol, vorzugsweise 300 bis 1000 mol Kohlenmonoxid bzw. die äquivalente Menge Kohlenmonoxid abspaltender Verbindung ein. Die Umsetzung erfolgt bei Temperaturen von 50 bis 160 °C, insbesondere 90 bis 130 °C und üblicherweise bei Drücken von 10 bis 40 MPa, vorzugsweise 20 bis 30 MPa. Die Behandlungsdauer, d.h. der Zeitraum, in der die Extraktion des Rhodiums aus der organischen Phase mit Hilfe der wäßrigen Lösung des Komplexbildners beendet ist, beträgt 0,1 bis 10 Stunden, insbesondere 0,5 bis 2 Stunden.

Kohlenmonoxid kann in reiner Form, aber auch als Gemisch mit inerten Gasen, z.B. Synthesegas, eingesetzt werden.

Als Verbindung, die unter den Reaktionsbedingungen Kohlenmonoxid bildet, besonders geeignet ist Formaldehyd, vorzugsweise als wäßrige Lösung. Der Einsatz von wäßriger Formaldehydlösung hat den Vorteil,

daß drucklos gearbeitet werden kann.

Nach Beendigung der Reaktion läßt man abkühlen und trennt die organische und die wäßrige Phase voneinander. Durch Zusatz eines inerten Lösungsmittels, z.B. eines Kohlenwasserstoffs wie Toluol oder Xylol zum Reaktionsgemisch, läßt sich die Phasentrennung verbessern. Aus der abgetrennten organischen Phase können durch Wasserwäsche noch Restmengen Rhodium gewonnen werden.

Mit besonderem Erfolg wird das erfindungsgemäße Verfahren zur Abtrennung und Wiedergewinnung von Rhodium aus den Destillationsrückständen der Hydroformylierung von Ethylen und end- und innenständiger verzweigter Olefine wie i-Hepten, Diisobutylen, Tri- und Tetrapropylen oder dem unter der Bezeichnung Dimersol im Handel befindlichen Gemisch von $C_8$-Olefinen angewandt. Selbstverständlich kann das Verfahren auch auf die Hydroformylierung unverzweigter end- und mittelständiger Olefine angewendet werden, wobei in der Regel die absoluten Rhodium-Konzentrationen niedriger liegen.

Die wäßrige, Rhodium in hoher Konzentration enthaltende Phase kann entweder unmittelbar oder gereinigt und aufkonzentriert wieder als Katalysatorlösung eingesetzt werden. Es ist auch möglich, das Rhodium als in Wasser schwer- oder unlösliche Verbindung, z.B. als Rhodium-2-ethylhexanoat abzuscheiden und weiterzuverwenden.

In den folgenden Beispielen wird die Erfindung erläutert. Es ist selbstverständlich nicht beabsichtigt, sie auf diese speziellen Ausführungsformen zu beschränken.

Die Abkürzungen TPPTS bzw. TPPDS stehen für Triphenylphosphintrisulfonat, bzw. Triphenylphosphindisulfonat.

Beispiel 1

In einem 1 1-Glasautoklaven werden 200 g eines Destillationsrückstands aus der i-Hepten-Hydroformylierung mit einem Rhodiumgehalt von 328 ppm vorgelegt. Bei einer Innentemperatur von 27 bis 30°C wird unter Rühren ein Druck von etwa 0,4 MPa eingestellt. Danach heizt man innerhalb von etwa 30 Minuten auf 100°C auf und leitet während dieses Zeitraums 120 l Luft durch den Autoklaveninhalt. Weitere 190 l Luft werden innerhalb 1 Stunde bei 100°C durch die Reaktionslösung geleitet. Danach kühlt man auf Raumtemperatur und versetzt den Reaktorinhalt mit 50 g wäßriger TPPTS-Lösung, die 23,7 Gew.-% TPPTS und 4,88 Gew.-% TPPDS enthält sowie mit 50 g 37 %igem Formalin. Das Reaktionsgemisch wird anschließend bei 116 bis 122°C 1 Stunde gerührt.

Nach Abkühlen auf 80°C setzt man 50 g Toluol zu und rührt 5 Minuten. Darauf trennt man die Phasen und extrahiert die organische Phase nochmals mit 50 g Wasser. Die organische Phase (257 g) enthält noch 9 ppm Rhodium, entsprechend einer Rhodiumabtrennung von 96,5 %.

Beispiel 2

Wie in Beispiel 1 beschrieben werden 200 g eines Destillationsrückstandes der i-Hepten-Hydroformylierung mit Luft behandelt. Danach gibt man 50 g TPPTS-Lösung (23,7 Gew.-% TPPTS und 4,88 Gew.-% TPPDS) zu und rührt das Reaktionsgemisch bei einer Temperatur von 120°C und einem Synthesegasdruck von 25 MPa eine Stunde. Nach Abkühlung, Zugabe von 180 g Toluol und Phasentrennung wird die verbleibende organische Phase zweimal mit je 50 g Wasser gewaschen. Die organische Phase (379 g) enthält noch 3 ppm Rhodium, entsprechend einer Rhodiumabtrennung von 98,3 %.

Beispiel 3 (Vergleich)

Es wird wie in Beispiel 1 verfahren, mit dem Unterschied, daß der Formalinzusatz unterbleibt. Der organische Rückstand (249 g) enthält 23,7 ppm Rhodium, entsprechend einer Rhodiumabtrennung von 91 %.

Beispiel 4

Es wird wie in Beispiel 1 verfahren, mit dem Unterschied, daß ein Destillationsrückstand der i-Octen-Hydroformylierung mit einem Rhodiumgehalt von 148 ppm eingesetzt wird. Der organische Rückstand (258 g) enthält noch 4,9 ppm Rhodium, entsprechend einer Rhodiumabtrennung von 95,7 %.

Beispiel 5

Es wird wie in Beispiel 2 verfahren, mit dem Unterschied, daß ein Destillationsrückstand der i-Octen-Hydroformylierung mit einem Rhodiumgehalt von 148 ppm eingesetzt wird. Der organische Rückstand (377 g)

enthält noch 0,8 ppm Rhodium, entsprechend einer Rhodiumabtrennung von 99 %.

Beispiel 6 (Vergleich)

Es wird wie in Beispiel 3 verfahren, mit dem Unterschied, daß ein Destillationsrückstand der i-Octen-Hydroformylierung mit einem Rhodiumgehalt von 148 ppm eingesetzt wird. Der organische Rückstand (247 g) enthält 11 ppm, entsprechend einer Rhodiumabtrennung von 90,8 %.

## Patentansprüche

1. Verfahren zur Abtrennung und Wiedergewinnung von Rhodium aus den Destillationsrückständen von Oxosyntheseprodukten dadurch gekennzeichnet, daß man die Destillationsrückstände zunächst mit einem Oxidationsmittel und darauf in Gegenwart von Kohlenmonoxid oder einer Kohlenmonoxid abspaltenden Verbindung mit der wäßrigen Lösung eines mit Rhodium eine wasserlösliche Komplexverbindung bildenden Reagenzes umsetzt.

2. Verfahren nach Anspruch 1 dadurch gekennzeichnet, daß man die Destillationsrückstände mit Sauerstoff oder einem Sauerstoff enthaltenden Gas behandelt.

3. Verfahren nach Anspruch 1 oder 2 dadurch gekennzeichnet, daß die Behandlung der Destillationsrückstände mit einem Oxidationsmittel bei 20 bis 200 °C, insbesondere 50 bis 150 °C, vorzugsweise 80 bis 120 °C erfolgt.

4. Verfahren nach einem oder mehreren der Ansprüche 1 bis 3 dadurch gekennzeichnet, daß die Behandlung mit dem Oxidationsmittel bei Drücken von 0,1 bis 5 MPa erfolgt.

5. Verfahren nach einem oder mehreren der Ansprüche 1 bis 4 dadurch gekennzeichnet, daß als komplexbildendes Reagenz organische Phosphine der allgemeinen Formen

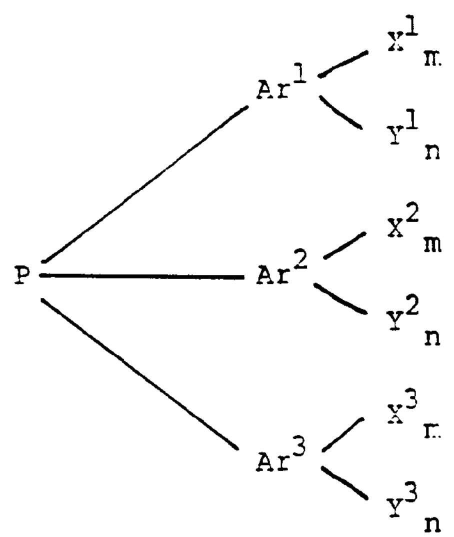

wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine geradkettige oder verzweigte Alkoxygruppe mit 1 bis 4 C-Atomen, ein Halogenatom, die OH-, CN-, $NO_2$-oder $R^1R^2N$-Gruppe, in der $R^1$ und $R^2$ für jeweils eine geradkettige oder verzweigte Alkylgruppe mit 1 bis 4 C-Atomen stehen, bedeuten, $X^1$, $X^2$ und $X^3$ ein Carboxylat-($COO^-$-) und/oder eine Sulfonat-($SO_3^-$-) Rest ist, $m_1$, $m_2$, $m_3$ gleiche oder verschiedene ganze Zahlen von 0 bis 3 sind, wobei mindestens eine Zahl $m_1$, $m_2$, $m_3$ gleich oder größer als 1 ist und $n_1$, $n_2$, $n_3$ gleiche oder verschiedene ganze Zahlen von 0 bis 5 sind.

6. Verfahren nach Anspruch 5 dadurch gekennzeichnet, daß $Ar^1$, $Ar^2$, $Ar^3$ jeweils einen Phenylrest, $X^1$, $X^2$, $X^3$ jeweils einen Sulfonatrest und $m_1$, $m_2$, $m_3$ jeweils die Zahl 0 bis 1 bedeuten, wobei die Summe von $m_1$, $m_2$, $m_3$ 1, 2 oder 3 ist.

7. Verfahren nach einem oder mehreren der Ansprüche 1 bis 6 dadurch gekennzeichnet, daß die mit einem Oxidationsmittel behandelten Destillationsrückstände mit Kohlenmonoxid und dem eine wasserlösliche Komplexverbindung bildenden Reagenz bei 50 bis 160 °C, vorzugsweise 90 bis 130 °C und bei Drücken von 10 bis 40 MPa, vorzugsweise 20 bis 30 MPa umgesetzt werden.

8. Verfahren nach einem oder mehreren der Ansprüche 1 bis 7 dadurch gekennzeichnet, daß die oxidierten Destillationsrückstände mit Formaldehyd als Kohlenmonoxid abspaltender Verbindung und dem eine wasserlösliche Komplexverbindung bildenden Reagenz bei 50 bis 160 °C, vorzugsweise 90 bis 130 °C behandelt werden.

9. Verfahren nach Anspruch 8 dadurch gekennzeichnet, daß Formaldehyd als wäßrige Lösung eingesetzt wird.

**Claims**

1. A process for separating and recovering rhodium from the distillation residues of Oxo synthesis products, characterised in that the distillation residues are first reacted with an oxidant and then, in the presence of carbon monoxide or a compound splitting off carbon monoxide, with the aqueous solution of a reagent forming a water-soluble complex compound with rhodium.

2. A process according to claim 1, characterised in that the distillation residues are treated with oxygen or a gas containing oxygen.

3. A process according to claim 1 or 2, characterised in that the distillation residues are treated with an oxidant at 20 to 200°C, in particular 50 to 150°C, preferably 80 to 120°C.

4. A process according to one or more of the claims 1 to 3, characterised in that the treatment with the oxidant takes place at pressures of 0.1 to 5 MPa.

5. A process according to one or more of the claims 1 to 4, characterised in that the complexing reagent is organic phosphines of the general formula:

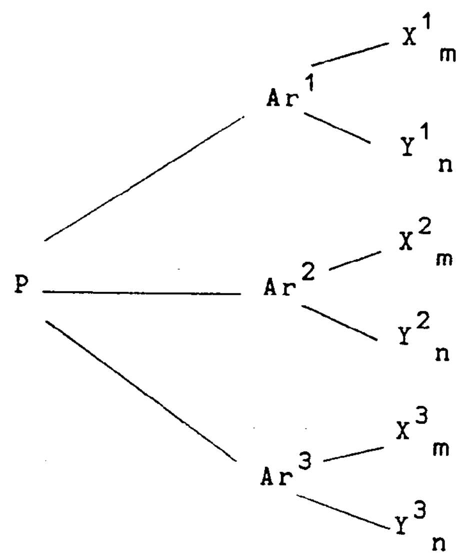

where $Ar^1$, $Ar^2$, $Ar^3$ are each a phenyl or naphthyl group, $Y^1$, $Y^2$, $Y^3$ each denote a straight-chain or branched alkyl group with 1 to 4 carbon atoms, a straight-chain or branched alkoxy group with 1 to 4 carbon atoms, a halogen atom, the OH, CN, $NO_2$ or $R^1R^2N$ group in which $R^1$ and $R^2$ each stand for a straight-chain or branched alkyl group with 1 to 4 carbon atoms, $X^1$, $X^2$ and $X^3$ are each a carboxylate($COO^-$) and/or a sulfonate($SO_3^-$) group, $m_1$, $m_2$, $m_3$ are the same or different integers from 0 to 3, at least one number $m_1$, $m_2$ or $m_3$ being equal to or greater than 1 and $n_1$, $n_2$, $n_3$ being the same or different integers from 0 to 5.

6. A process according to claim 5, characterised in that $Ar^1$, $Ar^2$, $Ar^3$ each denote a phenyl group, $X^1$, $X^2$, $X^3$ each stand for a sulfonate group and $m_1$, $m_2$, $m_3$ each denote the number 0 or 1, the sum of $m_1$, $m_2$ and $m_3$ being 1, 2 or 3.

7. A process according to one or more of the claims 1 to 6, characterised in that the distillation residues treated with an oxidant are reacted with carbon monoxide and the reagent forming a water-soluble complex compound at temperatures of 50 to 160°C, preferably 90 to 130°C and pressures of 10 to 40 MPa, preferably 20 to 30 MPa.

8. A process according to one or more of the claims 1 to 7, characterised in that the oxidised distillation residues are treated with formaldehyde as the compound which splits off carbon monoxide and with the reagent forming a water-soluble complex compound at 50 to 160°C, preferably 90 to 130°C.

9. A process according to claim 8, characterised in that the formaldehyde is used as an aqueous solution.

**Revendications**

1. Procédé pour la séparation et la récupération de rhodium de résidus de distillation de produits de synthèse oxo, caractérisé en ce que l'on fait réagir les résidus de distillation d'abord avec un agent oxydant et ensuite avec une solution aqueuse d'un réactif formant avec le rhodium un composé complexe soluble dans l'eau, en présence de monoxyde de carbone ou d'un composé libérant du monoxyde de carbone.

2. Procédé selon la revendication 1, caractérisé en ce que l'on traite le résidu de distillation par l'oxygène ou par un gaz contenant de l'oxygène.

3. Procédé selon la revendication 1 ou 2, caractérisé en ce que le traitement des résidus de distillation par un agent oxydant a lieu à 20-200°C, en particulier 50-150°C, de préférence 80-120°C.

4. Procédé selon une ou plusieurs des revendications 1 à 3, caractérisé en ce que le traitement par l'agent oxydant a lieu sous des pressions de 0,1 à 5 MPa.

5. Procédé selon une ou plusieurs des revendications 1 à 4, caractérisé en ce que l'on utilise comme réactif complexant des phosphines organiques de formule générale :

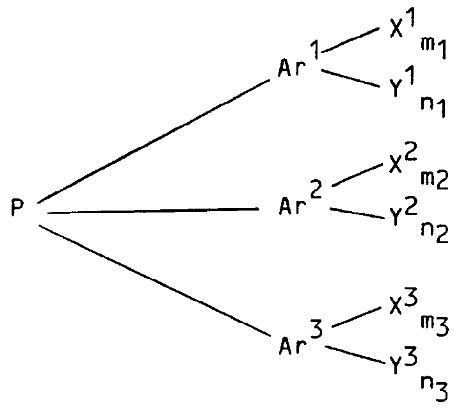

dans laquelle $Ar^1$, $Ar^2$ et $Ar^3$ représentent chacun un groupe phényle ou naphtyle, $Y^1$, $Y^2$ et $Y^3$ représentent chacun un groupe alkyle à chaîne droite ou ramifiée en $C_1$-$C_4$, un groupe alcoxy à chaîne droite ou ramifiée en $C_1$-$C_4$, un atome d'halogène, le groupe OH, CN, $NO_2$ ou $R^1R^2N$ dans lequel $R^1$ et $R^2$ représentent chacun un groupe alkyle à chaîne droite ou ramifiée en $C_1$-$C_4$, $X^1$, $X^2$ et $X^3$ représentent chacun un reste carboxylate ($COO^-$) et/ou un reste sulfonate ($SO_3^-$), $m_1$, $m_2$ et $m_3$ sont des nombres entiers identiques ou différents de 0 à 3, au moins un des nombres $m_1$, $m_2$ et $m_3$ étant égal ou supérieur à 1 et $n_1$, $n_2$ et $n_3$ sont des entiers identiques ou différents de 0 à 5.

6. Procédé selon la revendication 5, caractérisé en ce que $Ar^1$, $Ar^2$ et $Ar^3$ représentent chacun un reste phényle, $X^1$, $X^2$ et $X^3$ représentent chacun un reste sulfonate et $m_1$, $m_2$, $m_3$ sont chacun un nombre égal à 0 ou 1, la some de $m_1$, $m_2$ et $m_3$ étant de 1, 2 ou 3.

7. Procédé selon une ou plusieurs des revendications 1 à 6, caractérisé en ce que les résidus de distillation traités par un agent oxydant sont mis à réagir avec le monoxyde de carbone et le réactif formant un complexe soluble dans l'eau à 50-160°C, de préférence 90-130°C et sous des pressions de 10-40 MPa, de préférence 20-30 MPa.

8. Procédé selon une ou plusieurs des revendications 1 à 7, caractérisé en ce que les résidus de distillation oxydés sont traités par le formaldéhyde comme composé libérant du monoxyde de carbone et le réactif formant un composé complexe soluble dans l'eau à 50-160°C, de préférence 90-130°C.

9. Procédé selon la revendication 8, caractérisé en ce que le formaldéhyde est utilisé en solution aqueuse.